# FASCICULE DE BREVET EUROPEEN

(11) **EP 1 282 394 B1**
(45) Date de publication et mention de la délivrance du brevet: **29.09.2004**
(21) Numéro de dépôt: 01934113.0
(22) Date de dépôt: 14.05.2001
(51) Int. Cl.: A61K 7/40, A61K 7/48, A61P 17/16

(54) **UTILISATION DE DERIVES DE L'ACIDE N,N'-DIBENZYL ETHYLENE DIAMINE N,N'-DIACETIQUE COMME AGENT ANTI-POLLUTION**
VERWENDUNG VON N,N'-DIBENZYLETHYLENDIAMINDIESSIGSÄUREDERIVATEN ZUM SCHUTZ GEGEN VERSCHMUTZUNG
USE OF N,N'-DIBENZYL ETHYLENE DIAMINE N,N'-DIACETIC ACID DERIVATIVES AS ANTI-POLLUTION AGENT

(30) Priorité: 18.05.2000 FR 0006381
(43) Date de publication de la demande: 12.02.2003
(73) Titulaire: L'OREAL, 75008 Paris (FR)
(72) Inventeur: CATROUX, Philippe, F-94130 Nogent sur Marne (FR); COTOVIO, José, F-77270 Danmartin en Go[le (FR); DUCHE, Daniel, F-75013 Paris (FR); GALEY, Jean-Baptiste, F-93600 Aulnay sous Bois (FR)
(74) Mandataire: Casalonga, Axel
(86) Numéro de dépôt international: PCT/FR2001/001452
(87) Numéro de publication internationale: WO 2001/087253

(56) Documents cités:
- EP-A- 0 557 042
- DE-A- 19 601 060
- DE-A- 19 802 539
- FR-A- 2 768 145
- GB-A- 2 333 705
- US-A- 4 885 156
- US-A- 5 683 684
- US-A- 5 703 095
- US-A- 5 834 518
- J.-B. GALEY ET AL: "Protection against oxidative damage by iron chelators: Effect of lipophilic analogues and prodrugs of N,N'-Bis(3,4,5-trimethoxybenzyl)ethylenedi amine-N,N-diacetic acid" JOURNAL OF MEDICINAL CHEMISTRY, vol. 43, no. 7, mars 2000 (2000-03), pages 1418-1421, XP002161560 WASHINGTON US

## Description

La présente invention a pour objet l'utilisation en application topique de dérivés de l'acide N,N'-dibenzyl éthylène diamine N,N'-diacétique comme agent anti-pollution, tout comme un procédé de traitement cosmétique mettant en oeuvre lesdits dérivés.

Les ions métalliques sont nécessaires à l'organisme sous forme de traces comme nutriments essentiels. Par exemple, plusieurs fonctions impliquant des polypeptides telles que des fonctions enzymatiques, structurales et immunologiques requièrent des cofacteurs métalliques.

Cependant, d'autres ions métalliques, en particulier les ions des métaux lourds lorsqu'ils sont à des concentrations non physiologiques, peuvent altérer ces fonctions. Ainsi, la surexposition aux métaux de l'environnement peut conduire à des effets toxiques.

Des études écologiques conduites dans des pays industrialisés montrent que les quantités de métaux présents dans l'atmosphère sont croissantes. Ceci conduit à une augmentation des niveaux de métaux lourds dans les tissus des organismes consécutivement à l'ingestion de nourritures contaminées et à l'exposition aux métaux de l'atmosphère.

Les effets de l'accumulation des métaux lourds peuvent être extrêmement dangereux et leur toxicité est due en partie à l'altération des structures tertiaires et quaternaires des protéines, ce qui conduit à une réduction de leur activité catalytique. Les protéines altérées peuvent devenir antigéniques et entraîner une réponse immunitaire. Elles sont alors reconnues par l'organisme comme des agents polypeptidiques étrangers et peuvent causer des réponses auto-immunes.

Un autre mécanisme responsable des effets toxiques des métaux est la substitution compétitive de co-facteurs physiologiques naturels par les métaux lourds à des concentrations non physiologiques. Ainsi, le contrôle des métaux lourds polluants dans l'atmosphère est essentiel pour prévenir des maladies en relation avec l'exposition aux métaux.

Du fait de la contamination croissante de l'environnement par les métaux lourds et de leur présence ubiquitaire dans l'écosystème, la peau, le cheveu et les muqueuses accessibles représentent la surface de contact la plus large et favorise donc l'accumulation des métaux et leur absorption ultérieure dans l'organisme.

Certains métaux et composés métalliques présents dans les fabrications industrielles, les produits chimiques, la bijouterie, les vêtements, les produits médicamenteux, les colorants et les produits d'entretien sont impliqués dans des réactions d'irritation primaire, des réactions d'allergie et de carcinogénicité au niveau du tissu cutané.

Les métaux particulièrement incriminés dans l'environnement sont le cuivre, le cobalt, le zinc, le manganèse, le mercure et le nickel, le plomb et le cadmium.

Le rash cutané causé par la dermatite aux métaux est un problème rencontré chez les gens exposés à des quantités élevées de certains ions métalliques. L'exposition au nickel dans l'environnement est largement du à l'usage fréquent de ce métal dans les articles les bijoux, les bracelets de montre et les boutons de vêtement. La sensibilisation au nickel avec le développement de dermatite est un hasard industriel dans certains métiers.

Par ailleurs, le dépôt de métaux sur le cheveu est un phénomène inévitable. Le cheveu est un absorbant fort pour les métaux. La fixation est tellement forte qu'une fois ces métaux fixés et capturés par les sites anioniques de la fibre kératinique, ils sont difficiles à elluer. Le degré de fixation des métaux sur le cheveu dépend généralement de plusieurs facteurs tels que la taille de la fibre, sa porosité, et le temps d'exposition. Les métaux comme le cuivre, le plomb et le fer peuvent interférer avec des traitements chimiques tels que la coloration et le permanentage du cheveu.

Certains produits cosmétiques contiennent des métaux comme le magnésium, le cuivre ou le fer. L'absorption de ces métaux par les fibres kératiniques peut interférer avec des traitements chimiques comme les colorations, le blanchiment ou les effets de permanente. Ces interférences peuvent conduire à des problèmes de colorations ou des précipitation comme il est expliqué dans le brevet Américain US-5,635,167.

Il a été montré que certains métaux lourds pénètrent dans la peau et s'accumulent (A.B.G. Landsdown. Critical Reviews in Toxicology. 1995, 25:397-462). A fortes concentrations ils peuvent induire des mécanismes d'oxydation sur les lipides membranaires, une cytotoxicité directe capable d'aboutir à une nécrose cellulaire et une alkylation des nucléophiles cellulaires pouvant être à l'origine de phénomènes de sensibilisation ou de carcinogénèse.

(S.J. Stochs and D. Bagchi. Free Radical Biology and Medecine. 1995, 18:321-336.; M. E. Figueiredo Pereira et coll., The Journal of Biological Chemistry.1998, 21:12703-12709,; N.L. Acan et coll.,1995, Biochemical and Molecular Medecine, 54:33-37.)

Ainsi, il existe un besoin de compositions qui permettent d'éviter les effets néfastes dus à ces polluants et qui permettent de protéger les matières kératiniques.

Des solutions ont déjà été envisagées dans le traitement cosmétiques et thérapeutiques. On a découvert que des composés avec des groupes souffrés se comportent comme des séquestrants de métaux lourds, comme les métallothionéines dans le brevet EP 0 557 042 A1 ou les composés amino-acides avec des groupements soufrés dans la demande de brevet EP 0 914 815 A1.

La demande de brevet GB 2333705 A mentionne l'utilisation d'acide d'éthylène diamine disuccinique dans des compositions pour le traitement des irritations de la peau par les métaux lourds.

Par ailleurs, la demande WO 94/11338 décrit l'utilisation de N-aryl méthylène éthylène diamine triacétate, N-aryl méthylène iminodiacétate ou N,N'-diaryl méthylène éthylène diamine acétate contre le stress oxydant, à savoir son utilisation thérapeutique pour le traitement de cancers, d'états inflammatoires, d'ischémie/reperfusion, de maladies du système nerveux, et son utilisation contre le vieillissement.

Plus récemment, des dérivés de l'acide N,N'-dibenzyl éthylène diamine N,N'-diacétique ont été utilisés comme agents dépigmentants de la peau (EP 0820763).

US-A-5 703 095 décrit l'utilisation de composés de formule (I) pour combattre le stress oxydatif (voir revendication 1).

Le problème posé est donc de protéger la peau contre les métaux et leur effets délétères rencontrés dans la pollution urbaine en sélectionnant des principes actifs qui soient efficaces pour lutter contre ces effets et/ou empêcher la pénétration des métaux dans la peau.

Il a maintenant été constaté, de façon tout à fait surprenante, que l'utilisation de dérivés de l'acide N,N'-dibenzyl éthylène diamine N,N'-diacétique permettait de protéger les matières kératiniques des effets des polluants.

L'acide N,N'-dibenzyl éthylène diamine N,N'-diacétique présente moins d'effet secondaires que d'autres molécules complexantes comme la déferoxamine, l'EHPG, le DTPA. De plus, cette molécule possède des capacités anti-oxydantes.

Par conséquent, cette molécule permet de protéger les matières kératiniques des effets des polluants et de former avec le fer des complexes avec une faible constante d'association, ce qui diminue les risques toxicologiques liés a une perturbation du métabolisme cellulaire.

L'application topique de la molécule sous forme non esterifiée permet de conserver l'actif en situation extracellulaire, ce qui permet d'une part de limiter les risques d'interaction avec les ions divalents intracellulaires et d'autres part d'éviter une concentration par chélation des métaux provenant de la pollution dans la cellule.

Ces dérivés permettent une meilleure régénération cellulaire, tout comme un retour à une homéostasie, permettant ainsi d'obtenir des matières kératiniques plus saines.

Ainsi, l'invention a pour : objet principal l'utilisation en application topique d'au moins un dérivé de l'acide N,N'-dibenzyl éthylène diamine N,N'-diacétique comme agent anti-pollution, préférentiellement comme agent cosmétique anti-pollution.

On entend par agent cosmétique anti-pollution un agent qui protège la peau et les matières kératiniques de façon à prévenir, atténuer et/ou supprimer les effets délétères des métaux.

Ces dérivés peuvent notamment correspondre à ceux de formule (I) : 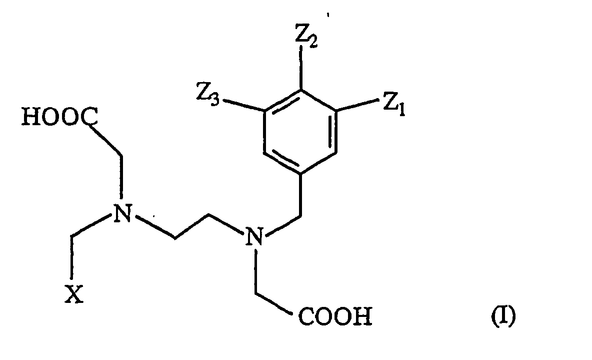 dans laquelle X représente COOH ou un cycle 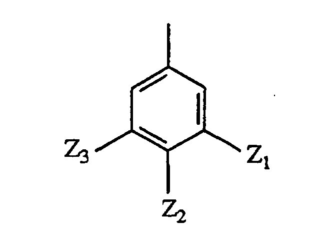 où Z₁, Z₂, Z₃, indépendamment les uns des autres, peuvent être H, OR ou R, R représentant un radical alkyle en C₁-C₈, linéaire ou ramifié, saturé ou insaturé, éventuellement substitué, ou un sel, un complexe métallique ou un ester d'un tel dérivé.

Le radical alkyle en C₁-C₈ est de préférence un radical saturé en C₁-C₄, tels que méthyle, éthyle, isopropyle et tert-butyle.

Comme substituant possible, on peut citer par exemple le groupe hydroxyle et les halogènes.

Comme sels, on peut citer les sels d'addition avec un acide minéral ou organique comme les acides sulfurique, chlorhydrique, nitrique, phosphorique ou acétique, et les sels d'addition avec une base minérale ou organique comme la soude, la potasse ou la triéthanolamine.

Comme complexes métalliques, on peut citer les complexes formés par addition de ZnCl₂ ou CaCl₂, par exemple.

Comme esters, on peut citer notamment l'ester méthylique ou l'ester éthylique.

Les composés utilisés dans la présente invention peuvent être préparés selon les méthodes décrites dans WO 94/11338.

De façon préférentielle, le dérivé de formule (I) est choisi parmi l'acide N,N'-di-(3-hydroxybenzyle)-éthylène diamine N,N'-diacétique et l'acide N,N'-di-(3,4,5-triméthoxybenzyle)-éthylène diamine N,N'-diacétique.

Selon l'invention,les dérivés de formule (I) sont utilisés afin de protéger les matières kératiniques des effets des métaux ou éléments métalloïdes polluants.

Dans le cadre de la présente invention, on entend par matières kératiniques notamment la peau, le cuir chevelu, les cheveux, les cils, les sourcils, les ongles et les muqueuses.

Les dérivés de formule (I) sont utilisés pour leur effet cytoprotecteur des matières kératiniques vis-à-vis des métaux ou éléments métalloïdes polluants.

Ils sont encore utilisés comme agents anti-polluants pour augmenter la régénération cellulaire et un retour à une homéostasie au niveau des matières kératiniques, afin d'obtenir des matières kératiniques plus saines.

L'invention a encore pour objet l'utilisation d'au moins un dérivé de formule (I) telle que définie ci-dessus ou un sel, un complexe métallique ou un ester d'un tel dérivé, dans ou pour la préparation d'une composition, préférentiellement cosmétique, à application topique anti-polluante.

Il a été constaté que les compositions utilisées selon l'invention présentent en outre de bonnes propriétés de stabilité et ne présentent pas de problèmes de toxicité liés a une perturbation du métabolisme cellulaire.

La composition anti-polluante conforme à l'invention peut contenir de 0,005% à 10% et de préférence de 0,1 à 5% en poids de composé de formule (I) par rapport au poids total de la composition.

La composition utilisée dans l'invention peut contenir en outre au moins un autre composé anti-pollution.

Ce dernier est notamment choisi parmi les anthocyanes et/ou ses dérivés, les composés contenant une fonction thio-éther, sulfoxide ou sulfone, l'ergothionéine et/ou ses dérivés, les chélateurs de métaux comme, les antioxydants, les extraits cellulaires de végétal de la famille des Pontederiaceae. Parmi les antioxydants, on choisira plus particulièrement les polyphénols et entre autre l'acide ellagique.

La composition utilisée dans l'invention, de préférence cosmétique, peut contenir en outre un milieu cosmétiquement acceptable qui est plus particulièrement constitué d'eau et/ou éventuellement de solvant organique cosmétiquement acceptable. Les solvants organiques peuvent représenter de 5 à 98% du poids total de la composition. Ils peuvent être choisis dans le groupe constitué par les solvants organiques hydrophiles, les solvants organiques lipophiles, les solvants amphiphiles ou leurs mélanges. Parmi les solvants organiques hydrophiles, on peut citer par exemple des alcools mono- ou polyfonctionnels tels que des mono-alcools inférieurs, linéaires ou ramifiés, ayant de 1 à 8 atomes de carbone comme l'éthanol, le propanol, le butanol, l'isopropanol, l'isobutanol, les polyéthylèneglycols éventuellement oxyéthylénés ayant de 6 à 80 oxydes d'éthylène, les polyols tels que le propylèneglycol, l'isoprène glycol, le butylèneglycol, le glycérol, le sorbitol et ses dérivés, les mono- ou dialkyles d'isosorbide dont les groupements alkyle ont de 1 à 5 atomes de carbone comme le diméthyl isosorbide, les éthers de glycol comme le diéthylène glycol mono-méthyle ou mono-éthyléther et les éthers de polypropylène glycol comme le dipropylène glycol méthyléther. Comme solvants organiques lipophiles, on peut citer par exemple les esters gras tels que l'adipate de diisopropyle, l'adipate de dioctyle, les benzoates d'alkyle. Comme solvants organiques amphiphiles, on peut citer des polyols tels que des dérivés de propylèneglycol (PPG), tels que les esters de polypropylèneglycol et notamment les esters de polypropylèneglycol et d'acides gras, de PPG et d'alcools gras comme le PPG-23 oleyléther et le PPG-36 oléate.

Afin que les compositions cosmétiques ou dermatologiques de l'invention soient plus agréables à utiliser (plus douces à l'application, plus nourrissantes, plus émolliantes), il est possible d'ajouter une phase grasse dans le milieu de ces compositions.

La phase grasse représente, de préférence, de 0 à 50% en poids total de la composition.

Cette phase grasse peut comporter une ou plusieurs huiles choisies de préférence dans le groupe constitué par :
- les silicones volatiles ou non-volatiles, linéaires, ramifiées ou cycliques, organo-modifiées ou non, hydrosolubles ou liposolubles,
- les huiles minérales telles que l'huile de paraffine et de vaseline,
- les huiles d'origine animale telles que le perhydrosqualène,
- les huiles d'origine végétale telles que l'huile d'amandes douces, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de Purcellin, les isoparaffines,
- les huiles fluorées et perfluorées,
- les esters d'acides gras tels que l'huile de Purcellin.

Elle peut aussi comporter comme matières grasses (un) ou plusieurs alcools gras, acides gras ou cires (paraffine, cire de polyéthylène, Carnauba, cire d'abeilles).

De façon connue, toutes les compositions de l'invention peuvent en outre contenir des adjuvants habituels dans le domaine cosmétique et dermatologique tels que les gélifiants et/ou épaississants classiques aqueux ou lipophiles, les actifs hydrophiles ou lipophiles, les conservateurs, des antioxydants, les parfums, les émulsionnants, les agents hydratants, les agents pigmentants, les dépigmentants, les agents kératolytiques, les vitamines, les émollients, les séquestrants, les tensio-actifs, les polymères, les agents alcalinisants ou acidifiants, les charges, les agents anti-radicaux libres, les céramides, les filtres solaires, notamment ultra-violets, les répulsifs pour insectes, les agents amincissants, les matières colorantes, les bactéricides, les antipelliculaires.

Les quantités de ces différents adjuvants sont celles classiquement utilisées dans les domaines considérés.

Bien entendu, l'homme de l'art veillera à choisir le ou les éventuels composés à ajouter à la composition selon l'invention, de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes galléniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules, ces sphérules pouvant être des nanoparticules polymériques telles que les nanosphères et les nanocapsules, ou mieux, des vésicules lipidiques de type ionique et/ou non-ionique.

Les compositions selon l'invention peuvent être plus ou moins fluides et avoir l'aspect d'une crème blanche ou colorée, d'une pommade, d'un lait, d'une lotion, d'un sérum, d'une pâte, d'une mousse.

Elles peuvent éventuellement être appliquées sur la peau sous forme d'aérosol.

Elles peuvent également se présenter sous forme solide, et par exemple sous forme de stick.

Elles peuvent être utilisées comme produit de soin et/ou comme produit de maquillage.

Les compositions de l'invention peuvent avoir un pH compris entre 3 et 8, préférentiellement entre 5 et 7.

Un autre objet de l'invention consiste en un procédé de traitement cosmétique destiné à obtenir une protection de l'organisme contre les effets de la pollution, consistant à appliquer sur la matière kératinique une quantité cosmétiquement efficace d'au moins un dérivé de formule (I) tel que défini ci-dessus.

Un autre procédé de traitement cosmétique selon l'invention, destiné à obtenir une protection de l'organisme contre les effets de la pollution, consiste à appliquer sur la matière kératinique une composition cosmétique selon l'invention telle que définie ci-dessus.

Les exemples qui suivent sont destinés à illustrer l'invention, sans pour autant présenter un caractère limitatif.

### EXEMPLEI

### Effet protecteur de l'acide N,N'-bis-(3,4,5-triméthoxy-benzyl)éthylène diamine N,N'-diacétique

### I. Protocole

L'effet cytotoxique du cadmium sur kératinocytes humains en culture a été évalué par une technique de mesure de la viabilité cellulaire appelée test d'incorporation du rouge neutre (E. Borenfreund et J.A. Puerner. Tisuue Culture Methods. 1984, 9:7-9).

L'étude a été réalisée sur une culture monocouche de kératinocytes humains issus de plasties chirurgicales. Les cellules sont ensemencées à J-3 en boîtes de culture 96 puits à raison de 25 000 cellules/cm² dans 100 µl de milieu de culture (milieu défini sans sérum de veau, NR2, Biofluids). Les incubations sont réalisées en étuve à 37°C en atmosphère humide et enrichies à 5% de CO₂.

Les cellules sont traitées 24 heures par des concentrations croissantes (0, 10, 25, 50, 75, 100, 150 et 200 µM) de chlorure de cadmium (CdCl₂) seul, de façon à déterminer sa cytotoxicité. Parallèlement, on réalise un traitement dans les mêmes conditions, mais en présence d'acide N,N'-bis-(3,4,5-triméthoxy benzyl)éthylène diamine N,N'-diacétique (100 et 50 µM). Les incubations sont réalisées en étuve à 37°C, en atmosphère humide.

A la fin de la période de contact, on réalise un test d'incorporation de rouge neutre et on effectue une lecture spectrophotométrique à 550 nm.

Pour ce faire, on rince les cellules par du tampon PBS afin d'éliminer les solutions de traitement; on ajoute 100 µl par puits d'une solution à 0,5 mg/ml de rouge neutre dans le milieu de culture; on incube à 37°C 5% de CO₂, sous atmosphère humide, pendant 3 heures; on rince au PBS; on fixe avec une solution de formol/calcium, pendant 1 minute; on extrait le rouge neutre par 100 µl/puits d'une solution d'éthanol-acide acétique; on lit la densité optique au spectrophotomètre à 550 nm et on calcule la concentration de CdCl₂, entraînant une chute de 50% de la viabilité = CI-50.

### II. Résultats

Les résultats sont des résultats moyens. On a effectué 3 expériences indépendantes et on a effectué 4 mesures par expérience.

Les résultats donnés en concentration provoquant une chute de 50% de la viabilité cellulaire sont repris dans le tableau suivant:

Cytotoxicité du chlorure de cadmium envers les kératinocytes humains en culture, en présence ou en absence d'acide N,N'-diméthyl éthylène diamine N,N'-diacétique, à deux concentrations: 50 µM et 100 µM.

| **CI.50 du chlorure de cadmium** | | |
|---|---|---|
| **sans protecteur** | **avec protecteur** | |
| Moyenne +/- SEM | Moyenne +/- SEM | |
| | 50 µM | 100 µM |
| | | |
| 39 +/- 1,15 µM | 92 +/- 7,5µM | 122 +/- 4,6 µM |

Le chlorure de cadmium seul présente une toxicité importante avec une CI.50 de 39µM. En présence d'acide N,N'-diméthyl éthylène diamine N,N'-diacétique, la cytotoxicité du chlorure de cadmium diminue significativement (ce qui correspond à une augmentation de la CI.50).
- à 50 µM de protecteur, la cytotoxicité diminue d'un facteur 2,3.
- à 100 µM de protecteur, la cytotoxicité diminue d'un facteur 3,1.

### III. Conclusion

A partir d'un modèle biologique in vitro, il a été démontré qu'un agent représentatif d'une catégorie de polluants atmosphériques comme le cadmium, entraîne dans les conditions expérimentales une forte toxicité, et que l'acide N,N'-bis-(3,4,5-triméthoxybenzyl)éthylène diamine N,N'-diacétique protège de façon importante les cellules contre la toxicité de ce polluant.

### EXEMPLES DE FORMULATION

### Exemple 1:

Selon les techniques usuelles de préparation, on mélange les constituants ci-dessous pour préparer une émulsion.

| | |
|---|---|
| l'acide N,N'-di-(3,4,5-triméthoxybenzyle)-éthylène diamine N,N'- diacétique | 1 g |
| polyéthylèneglycol oxyéthyléné par 50 moles d'oxyde d'éthylène | 3 g |
| monodiglycérylstéarate | 3 g |
| huile de vaseline | 24 g |
| alcool cétylique | 5 g |
| eau | qsp 100 g |

### Exemple 2:

De la même manière, on prépare une émulsion selon une technique classique, à partir des composés suivants:

| | |
|---|---|
| l'acide N,N'-di-(3-hydroxybenzyle)-éthylène diamine N,N'-diacétique | 0,1 g |
| octylpalmitate | 10 g |
| glycérylisostéarate | 4 g |
| huile de vaseline | 24 g |
| vitamine E | 1 g |
| glycérol | 3 g |
| eau | qsp 100 g |

### Exemple 3:

A partir des constituants ci-dessous, on formule la composition suivante:

| | |
|---|---|
| l'acide N,N'-di-(3-hydroxybenzyle)-éthylène diamine N,N'-diacétique | 2 g |
| huile de jojoba | 13 g |
| parabenzoxy benzoate de méthyle et d'isopropyle | 0,05 g |
| sorbate de potassium | 0,3 g |
| cyclopentadimethylsiloxane | 10 g |
| alcool stéarylique | 1 g |
| acide stéarique | 4 g |
| stéarate de polyéthylèneglycol | 3 g |
| vitamine E | 1 g |
| glycérol | 3 g |
| eau | qsp 100 g |

### Exemple 4:

A partir des constituants ci-dessous, on formule la composition suivante:

| | |
|---|---|
| l'acide N,N'-di-(3-hydroxybenzyle)-éthylène diamine N,N'-diacétique | 1,5 g |
| huile de paraffine | 13 g |
| parabenzoxy benzoate de méthyle et d'isopropyle | 0,05 g |
| sorbate de potassium | 0,3 g |
| cyclopentadimethylsiloxane | 10 g |
| acide ellagique | 0,1 g |
| alcool stéarylique | 1 g |
| acide stéarique | 4 g |
| stéarate de polyéthylèneglycol | 3 g |
| vitamine E | 1 g |
| glycérol | 3 g |
| eau | qsp 100 g |

## Revendications

1. Utilisation dans la préparation d'un agent topique de protection de la peau et des matières kératiniques de façon à prévenir, atténuer ou supprimer les effets délétères des métaux lourds, d'au moins un dérivé de formule (I) : 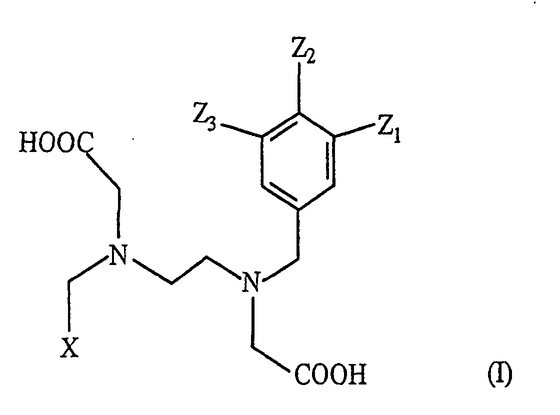 dans laquelle X représente COOH ou un cycle 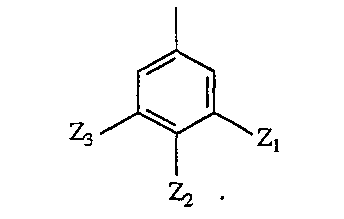 oùZ₁, Z₂, Z₃, indépendamment les uns des autres, peuvent être H, OR ou R, R représentant un radical alkyle en C₁-C₈, linéaire ou ramifié, saturé ou insaturé, éventuellement substitué,
ou un sel, un complexe métallique ou un ester d'un tel dérivé.

2. Utilisation cosmétique en application topique comme agent de protection de la peau et des matières kératiniques de façon à prévenir, atténuer ou supprimer les effets délétères des métaux lourds, d'au moins un dérivé de formule (I) telle que définie dans la revendication 1.

3. Utilisation telle que définie dans la revendication 1 d'au moins un dérivé de formule (I), dans la préparation d'un agent topique de protection de la peau et des matières kératiniques de façon à prévenir, atténuer ou supprimer les effets délétères des métaux lourds, présentant un effet cytoprotecteur des matières kératiniques vis-à-vis des métaux ou éléments métalloïdes polluants.

4. Utilisation cosmétique en application topique d'au moins un dérivé de formule (I) telle que définie dans la revendication 1, comme agent présentant un effet cytoprotecteur des matières kératiniques vis-à-vis des métaux ou éléments métalloïdes polluants.

5. Utilisation d'au moins un dérivé de formule (I) tel que défini dans la revendication 1 ou 3 dans la préparation d'un agent topique pour augmenter la régénération cellulaire et un retour à une hornéostasie au niveau des matières kératiniques, afin d'obtenir des matières kératiniques plus saines.

6. Utilisation cosmétique en application topique d'au moins un dérivé de formule (I) tel que défini dans la revendication 1 ou 3, pour augmenter la régénération cellulaire et un retour à une homéostasie au niveau des matières kératiniques, afin d'obtenir des matières kératiniques plus saines.

7. Utilisation selon l'une quelconque des revendications 1 à 5, **caractérisée en ce que** le dérivé de formule (I) est choisi parmi l'acide N,N'-di-(3-hydroxybenzyle)-éthylène diamine N,N'-diacétique et l'acide N,N'-di-(3,4,5-triméthoxybenzyle)-éthylène diamine N,N'- diacétique.

8. Utilisation d'un dérivé de formule (I) telle que définieselon l'une quelconque des revendications 1 à 7, dans la préparation d'une composition, topique de protection de la peau et des matières kératiniques de façon à prévenir, atténuer ou supprimer les effets délétères des métaux lourds.

9. Utilisation cosmétique d'un dérivé de formule (I) telle que défini selon l'une quelconque des revendications 1 à 7 dans une composition à application topique de protection de la peau et des matières kératiniques de façon à prévenir, atténuer ou supprimer les effets délétères des métaux lourds.

10. Utilisation selon la revendication 8, **caractérisée en ce que** ladite composition cosmétique anti-polluante contient de 0,005% à 10% et de préférence de 0,1 à 5% en poids de composé de formule (I) par rapport au poids total de la composition.

11. Utilisation selon la revendication 8 ou 10, **caractérisée en ce que** ladite composition contient en outre au moins un autre composé anti-pollution.

12. Utilisation selon la revendication 11, **caractérisée en ce que** ledit composé anti-pollution est choisi parmi les anthocyanes et/ou ses dérivés, les composés contenant une fonction thio-éther, sulfoxide ou sulfone, l'ergothionéine et/ou ses dérivés, les chélateurs de métaux comme, les antioxydants et notamment l'acide ellagique, les extraits cellulaires de végétal de la famille des Pontederiaceae.

13. Utilisation selon l'une quelconque des revendications 8 à 12, **caractérisée en ce que** la composition contient en outre un milieu cosmétiquement acceptable constitué d'eau et/ou d'au moins un solvant organique choisi dans le groupe constitué par les solvants organiques hydrophiles, les solvants organiques lipophiles, les solvants amphiphiles ou leurs mélanges.

14. Utilisation selon la revendication 13, **caractérisée en ce que** les solvants organiques sont choisis dans le groupe constitué par les alcools mono- ou polyfonctionnels, les polyéthylène glycols éventuellement oxyéthylénés, les esters de polypropylène glycol, le sorbitol et ses dérivés, les dialkyls d'isosorbide, les éthers de glycol et des éthers de polypropylène glycol, les esters gras.

15. Utilisation selon la revendication 13 ou 14, **caractérisée en ce que** le ou les solvants organiques représentent de 5 à 98% du poids total de la composition.

16. Utilisation selon l'une quelconque des revendications 8 à 15, **caractérisée en ce que** la composition comprend en outre au moins une phase grasse.

17. Utilisation selon la revendication 16, **caractérisée en ce que** la phase grasse représente de 0 à 50% du poids total de la composition.

18. Utilisation selon l'une quelconque des revendications 8 à 17, **caractérisée par le fait que** la composition contient en outre au moins un additif choisi dans le groupe constitué par les gélifiants et/ou épaississants classiques aqueux ou lipophiles, les actifs hydrophiles ou lipophiles, les conservateurs, des antioxydants, les parfums, les émulsionnants, les agents hydratants, les agents pigmentants, les dépigmentants, les agents kératolytiques, les vitamines, les émollients, les séquestrants, les tensio-actifs, les polymères, les agents alcalinisants ou acidifiants, les charges, les agents anti-radicaux libres, les céramides, les filtres solaires, notamment ultra-violets, les répulsifs pour insectes, les agents amincissants, les matières colorantes, les bactéricides, les antipelliculaires.

19. Utilisation selon l'une quelconque des revendications 8 à 18, **caractérisée en ce que** la composition se présente sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules.

20. Utilisation selon l'une quelconque des revendications 8 à 19, **caractérisée en ce que** la composition a l'aspect d'une crème blanche ou colorée, d'une pommade, d'un lait, d'une lotion, d'un sérum, d'une pâte, d'une mousse ou d'un solide.

21. Utilisation selon l'une quelconque des revendications 8 à 20, **caractérisée en ce que** la composition présente un pH compris entre 3 et 8, préférentiellement entre 5 et 7.

22. Procédé de traitement cosmétique destiné à obtenir une protection de l'organisme contre les effets de la pollution, **caractérisé en ce qu'**il consiste à appliquer sur la matière kératinique une quantité cosmétiquement efficace d'au moins un dérivé de formule (I) tel que défini dans la revendication 1 ou 7.

23. Procédé de traitement cosmétique destiné à obtenir une protection de l'organisme contre les effets de la pollution, **caractérisé en ce qu'**il consiste à appliquer sur la matière kératinique une composition cosmétique telle que définie dans l'une quelconque des revendications 8 à 21.

## Patentansprüche

1. Verwendung mindestens eines Derivats der Formel (I): 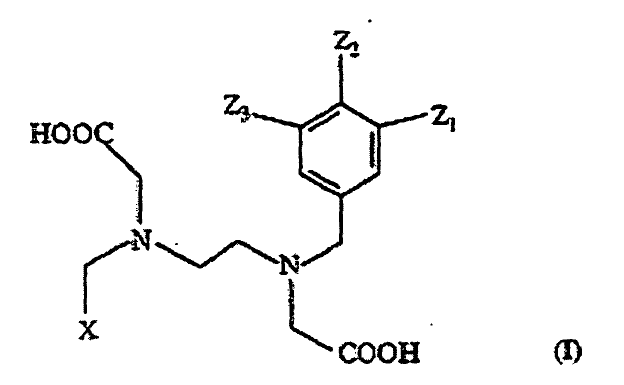 worin X COOH oder einen Ring 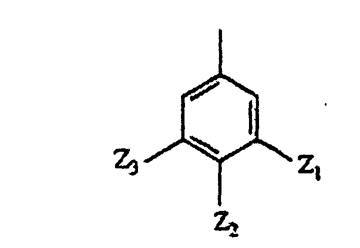 bedeutet, worin Z₁, Z₂ und Z₃ unabhängig voneinander H, OR oder R bedeuten können, wobei R eine geradkettige oder verzweigte, gesättigte oder ungesättigte, gegebenenfalls substituierte C₁₋₈-Alkylgruppe ist,
oder eines Salzes, Metallkomplexes oder Esters eines solchen Derivats zur Herstellung eines topischen Mittels zum Schutz der Haut und der Keratinsubstanzen, um den schädlichen Wirkungen von Schwermetallen vorzubeugen, sie abzuschwächen oder zu unterdrücken.

2. Kosmetische Verwendung mindesten seines Derivats der Formel (I) nach Anspruch 1 durch topische Anwendung als Mittel zum Schutz der Haut und der Keratinsubstanzen, um den schädlichen Wirkungen von Schwermetallen vorzubeugen, sie abzuschwächen oder zu unterdrücken.

3. Verwendung mindestens eines Derivats der Formel (I) nach Anspruch 1 als Mittel mit Zellschutzwirkung für Keratinsubstanzen gegenüber schädlichen Metallen oder Halbmetallen.

4. Kosmetische Verwendung mindestens eines Derivats der Formel (I) nach Anspruch 1 durch topische Anwendung als Wirkstoff mit Zellschutzwirkung für Keratinsubstanzen gegenüber schädlichen Metallen oder Halbmetallen.

5. Verwendung mindestens eines Derivats der Formel (I) nach Anspruch 1 oder 3 zur Verbesserung der Regeneration der Zellen und für die Rückkehr der Keratinsubstanzen zur Homöostase, um so gesündere Keratinsubstanzen zu erhalten.

6. Kosmetische Verwendung mindestens eines Derivats der Formel (I) nach Anspruch 1 oder 3 durch topische Anwendung, um die Regeneration der Zellen zu erhöhen und für eine Rückkehr der Keratinsubstanzen zur Homöostase, um schließlich gesündere Keratinsubstanzen zu erhalten.

7. Verwendung nach einem der Ansprüche 1 bis 5, **dadurch gekennzeichnet, dass** das Derivat der Formel (I) unter N,N'-Di-(3-Hydroxybenzyl)-ethylendiamin-N,N'-diessigsäure und N,N'-Di-(3,4,5-Trimethoxybenzyl)-ethylendiamin-N,N'-diessigsäure ausgewählt ist.

8. Verwendung eines Derivats der Formel (I) nach einem der Ansprüche 1 bis 7 zur Herstellung einer topischen Zusammensetzung zum Schutz der Haut und der Keratinsubstanzen, um den schädlichen Wirkungen von Schwermetallen vorzubeugen oder sie abzuschwächen oder zu unterdrücken.

9. Kosmetische Verwendung eines Derivats der Formel (I) und einem der Ansprüche 1 bis 7 in einer Zusammensetzung zur topischen Anwendung zum Schutz der Haut und der Keratinsubstanzen, um den schädlichen Wirkungen von Schwermetallen vorzubeugen oder sie abzuschwächen oder zu unterdrücken.

10. Verwendung nach Anspruch 8, **dadurch gekennzeichnet, dass** die gegen Umweltverschmutzung schützende kosmetische Zusammensetzung 0,005 bis 10 % und vorzugsweise 0,1 bis 5 Gew.-% der Verbindung der Formel (I), bezogen auf das Gesamtgewicht der Zusammensetzung, enthält.

11. Verwendung nach Anspruch 8 oder 10, **dadurch gekennzeichnet, dass** die Zusammensetzung ferner mindestens eine weitere Anti-Pollutions-Verbindung enthält.

12. Verwendung nach Anspruch 11, **dadurch gekennzeichnet, dass** diese gegen Umweltverschmutzung schützende Verbindung unter den Anthocyanen und/oder ihren Derivaten, Verbindungen, die eine Thioetherfunktion, Sulfoxidfunktion oder Sulfonfunktion enthalten, Ergothionein und/oder seinen Derivaten, Metallchelatbildnern, Antioxidantien und insbesondere Ellagsäure und Zellextrakten von Pflanzen aus der Familie der Pontederiaceae ausgewählt ist.

13. Verwendung nach einem der Ansprüche 8 bis 12, **dadurch gekennzeichnet, dass** die Zusammensetzung ferner ein kosmetisch akzeptables Medium enthält, das aus Wasser und/oder mindestens einem organischen Lösungsmittel besteht, das unter den hydrophilen organischen Lösungsmitteln, lipophilen organischen Lösungsmitteln, amphiphilen Lösungsmitteln und der Gemischen ausgewählt ist.

14. Verwendung nach Anspruch 13, **dadurch gekennzeichnet, dass** die organischen Lösungsmittel unter den mono- oder polyfunktionellen Alkoholen, gegebenenfalls ethoxylierten Polyethylenglykolen, Polypropylenglykolestern, Sorbit und seinen Derivaten, Dialkylisosorbiden, Glykolethern, Polypropylenglykolethern und Fettsäuren ausgewählt sind.

15. Verwendung nach Anspruch 13 oder 14, **dadurch gekennzeichnet, dass** das organische Lösungsmittel oder die organischen Lösungsmittel 5 bis 98 % des Gesamtgewichts der Zusammensetzung ausmachen.

16. Verwendung nach einem der Ansprüche 8 bis 15, **dadurch gekennzeichnet, dass** die Zusammensetzung ferner mindestens eine Fettphase enthält.

17. Verwendung nach Anspruch 16, **dadurch gekennzeichnet, dass** die Fettphase 0 bis 50 % des Gesamtgewichts der Zusammensetzung ausmacht.

18. Verwendung nach einem der Ansprüche 8 bis 17, **dadurch gekennzeichnet, dass** die Zusammensetzung ferner mindestens einen Zusatzstoff enthält, der unter den herkömmlichen wässrigen oder lipophilen Gelbildnern und/oder Verdickungsmitteln, hydrophilen oder lipophilen Wirkstoffen, Konservierungsmitteln, Antioxidantien, Parfums, Emulgatoren, Hydratisierungsmitteln, pigmentierenden Wirkstoffen, depigmentierenden Wirkstoffen, Keratolytika, Vitaminen, Emollientien, Maskierungsmitteln, grenzflächenaktiven Stoffen, Polymeren, Alkalisierungsmitteln, Ansäuerungsmitteln, Füllstoffen, Radikalfängern für freie Radikale, Ceramiden, Sonnenschutzfiltern und insbesondere UV-Filtern, Insekten abwehrenden Wirkstoffen, schlanker machenden Stoffen, Farbmitteln, Bakteriziden und Antischuppenmitteln ausgewählt sind.

19. Verwendung nach einem der Ansprüche 8 bis 18, **dadurch gekennzeichnet, dass** die Zusammensetzung als wässrige, wässrigalkoholische oder ölige Lösung, Öl-in-Wasser-Emulsion, Wasserin-Öl-Emulsion, multiple Emulsion, wässriges oder öliges Gel, flüssiges, pastöses oder festes wasserfreies Produkt oder Dispersion eines Öls in einer wässrigen Phase mithilfe von Kügelchen vorliegt.

20. Verwendung nach einem der Ansprüche 8 bis 19, **dadurch gekennzeichnet, dass** die Zusammensetzung wie eine weiße oder farbige Creme, eine Salbe, eine Milch, eine Lotion, ein Serum, eine Paste, ein Schaum oder ein Feststoff aussieht.

21. Verwendung nach einem der Ansprüche 8 bis 20, **dadurch gekennzeichnet, dass** die Zusammensetzung einen pH-Wert von 3 bis 8 und vorzugsweise 5 bis 7 aufweist.

22. Verfahren zur kosmetischen Behandlung, um den Organismus gegen die Wirkungen der Umweltverschmutzung zu schützen, **dadurch gekennzeichnet, dass** auf die Keratinsubstanz eine kosmetisch wirksame Menge mindestens eines Derivats der Formel (I) nach Anspruch 1 oder 7 aufgetragen wird.

23. Verfahren zur kosmetischen Behandlung zum Schutz des Organismus gegen die Wirkungen der Umweltverschmutzung, **dadurch gekennzeichnet, dass** auf die Keratinsubstanz eine kosmetische Zusammensetzung nach einem der Ansprüche 8 bis 21 aufgetragen wird.

## Claims

1. Use in the preparation of a topical agent for protecting the skin and keratin materials so as to prevent, attenuate or eliminate the deleterious effects of heavy metals of at least one derivative of formula (I): 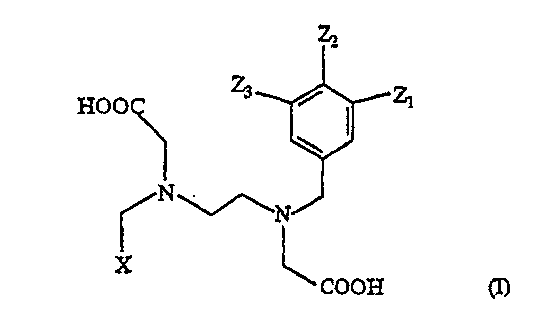 in which X represents COOH or a ring 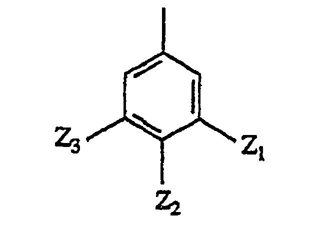 in which Z₁, Z₂ and Z₃, independently of each other, may be H, OR or R, R representing a linear or branched, saturated or unsaturated, optionally substituted C₁-C₈ alkyl radical,
or a salt, a metal complex or an ester of such a derivative.

2. Cosmetic use in topical application as an agent for protecting the skin and keratin materials so as to prevent, attenuate or eliminate the deleterious effects of heavy metals, of at least one derivative of formula (I) as defined in Claim 1.

3. Use as defined in Claim 1, of at least one derivative of formula (I) in the preparation of a topical agent for protecting the skin and keratin materials so as to prevent, attenuate or eliminate the deleterious effects of heavy metals with a cytoprotective effect on keratin materials with respect to pollutant elements or metalloid elements.

4. Cosmetic use in topical application of at least one derivative of formula (I) as defined in Claim 1, as agent with a cytoprotective effect on keratin materials with respect to pollutant metals or metalloid elements.

5. Use of at least one derivative of formula (I) as defined in Claim 1 or 3 in the preparation of a topical agent, for increasing cell regeneration and a return to homeostasis in keratin materials, in order to obtain healthier keratin materials.

6. Cosmetic use in topical application of at least one derivative of formula (I) as defined in Claim 1 or 3, for increasing cell regeneration and a return to homeostasis in keratin materials, in order to obtain healthier keratin materials.

7. Use according to any one of Claims 1 to 5, **characterized in that** the derivative of formula (I) is chosen from N,N'-bis(3-hydroxybenzyl)ethylenediamine-N,N'-diacetic acid and N,N'-bis(3,4,5-trimethoxybenzyl)ethylenediamine-N,N'-diacetic acid.

8. Use of a derivative of formula (I) as defined according to any one of Claims 1 to 7, in the preparation of a topical composition, for protecting the skin and keratin materials so as to prevent, attenuate or eliminate the deleterious effects of heavy metals.

9. Cosmetic use of a derivative of formula (I) as defined according to any one of Claims 1 to 7, in a composition for topical application for protecting the skin and keratin materials so as to prevent, attenuate or eliminate the deleterious effects of heavy metals.

10. Use according to Claim 8, **characterized in that** said antipolluting cosmetic composition contains from 0.005% to 10% and preferably 0.1% to 5% by weight of compound of formula (I) relative to the total weight of the composition.

11. Use according to Claim 8 or 10, **characterized in that** said composition also contains at least one other antipollution compound.

12. Use according to Claim 11, **characterized in that** said antipollution compound is chosen from anthocyans and/or derivatives thereof, compounds containing a thioether, sulphoxide or sulphone function, ergothioneine and/or its derivatives, metal-chelating agents such as antioxidants and in particular ellagic acid, and extracts of cells of a plant of the Pontederiacea family.

13. Use according to any one of Claims 8 to 12, **characterized in that** the composition also comprises a cosmetically acceptable medium consisting of water and/or of at least one organic solvent chosen from the group consisting of hydrophilic organic solvents, lipophilic organic solvents and amphiphilic solvents, or mixtures thereof.

14. Use according to Claim 13, **characterized in that** the organic solvents are chosen from the group consisting of mono- or polyfunctional alcohols, optionally oxyethylenated polyethylene glycols, polypropylene glycol esters, sorbitol and its derivatives, dialkyl isosorbides, glycol ethers and polypropylene glycol ethers, and fatty esters.

15. Use according to Claim 13 or 14, **characterized in that** the organic solvent(s) represent(s) from 5% to 98% relative to the total weight of the composition.

16. Use according to any one of Claims 8 to 15, **characterized in that** the composition also comprises at least one fatty phase.

17. Use according to Claim 16, **characterized in that** the fatty phase represents from 0 to 50% relative to the total weight of the composition.

18. Use according to any one of Claims 8 to 17, **characterized in that** the composition also contains at least one additive chosen from the group consisting of standard aqueous or lipophilic gelling agents and/or thickeners, hydrophilic or lipophilic active agents, preserving agents, antioxidants, fragrances, emulsifiers, moisturizers, pigmenting agents, depigmenting agents, keratolytic agents, vitamins, emollients, sequestering agents, surfactants, polymers, acidifying or basifying agents, fillers, free-radical scavengers, ceramides, sunscreens, especially ultraviolet screening agents, insect repellents, slimming agents, colorants, bactericides and antidandruff agents.

19. Use according to any one of Claims 8 to 18, **characterized in that** the composition is in the form of an aqueous, aqueous-alcoholic or oily solution, an oil-in-water or water-in-oil or multiple emulsion, an aqueous or oily gel, a liquid, pasty or solid anhydrous product or a dispersion of oil in an aqueous phase with the aid of spherules.

20. Use according to any one of Claims 8 to 19, **characterized in that** the composition has the appearance of a white or coloured cream, an ointment, a milk, a lotion, a serum, a paste, a mousse or a solid.

21. Use according to any one of Claims 8 to 20, **characterized in that** the composition has a pH of between 3 and 8 and preferably between 5 and 7.

22. Cosmetic treatment process for protecting the body against the effects of pollution, **characterized in that** it consists in applying to keratin material a cosmetically effective amount of at least one derivative of formula (I) as defined in Claim 1 or 7.

23. Cosmetic treatment process for protecting the body against the effects of pollution, **characterized in that** it consists in applying to keratin material a cosmetic composition as defined in any one of Claims 8 to 21.
